# EUROPEAN PATENT SPECIFICATION

(11) **EP 4 492 050 B1**
(45) Date of publication and mention of the grant of the patent: **25.02.2026**
(21) Application number: 22930999.2
(22) Date of filing: 27.10.2022
(51) Int. Cl.: G01N 27/62, C12Q 1/6872, G16B 15/00, G16B 30/00, G16B 40/10

(54) **NUCLEIC ACID STRUCTURE ANALYSIS METHOD AND NUCLEIC ACID STRUCTURE ANALYSIS DEVICE**
VERFAHREN ZUR ANALYSE EINER NUKLEINSÄURESTRUKTUR UND VORRICHTUNG ZUR ANALYSE EINER NUKLEINSÄURESTRUKTUR
PROCÉDÉ D'ANALYSE DE LA STRUCTURE D'UN ACIDE NUCLÉIQUE ET DISPOSITIF D'ANALYSE DE LA STRUCTURE D'UN ACIDE NUCLÉIQUE

(30) Priority: 08.03.2022 JP 2022035266
(43) Date of publication of application: 15.01.2025
(73) Proprietor: SHIMADZU CORPORATION, Kyoto-shi, Kyoto 604-8511 (JP)
(72) Inventor: YAMAUCHI, Shosei, Kyoto-shi, Kyoto 604-8511 (JP); TAKAHASHI, Hidenori, Kyoto-shi, Kyoto 604-8511 (JP)
(74) Representative: Müller-Boré & Partner Patentanwälte PartG mbB
(86) International application number: PCT/JP2022/040077
(87) International publication number: WO 2023/171024

(56) References cited:
- WO-A1-2015/133259
- JP-A- 2016 223 777
- SANTOS IN�S C. ET AL: "Recent developments in the characterization of nucleic acids by liquid chromatography, capillary electrophoresis, ion mobility, and mass spectrometry (2010-2020)", JOURNAL OF SEPARATION SCIENCE, vol. 44, no. 1, 15 October 2020 (2020-10-15), DE, pages 340 - 372, XP093281421, ISSN: 1615-9306, DOI: 10.1002/jssc.202000833
- JINGJIE MO ET AL: "Characterization of nucleic acid higher order structure by high-resolution tandem mass spectrometry", ANALYTICAL AND BIOANALYTICAL CHEMISTRY, SPRINGER, BERLIN, DE, vol. 386, no. 3, 20 July 2006 (2006-07-20), pages 675 - 681, XP019441114, ISSN: 1618-2650, DOI: 10.1007/S00216-006-0614-Z
- HIDENORI TAKAHASHI ET AL: "Hydrogen Attachment/Abstraction Dissociation (HAD) of Gas-Phase Peptide Ions for Tandem Mass Spectrometry", ANALYTICAL CHEMISTRY, vol. 88, no. 7, 22 March 2016 (2016-03-22), pages 3810 - 3816, XP055546231, DOI: 10.1021/acs.analchem.5b04888
- HIDENORI TAKAHASHI ET AL: "Structural Analysis of Phospholipid Using Hydrogen Abstraction Dissociation and Oxygen Attachment Dissociation in Tandem Mass Spectrometry", ANALYTICAL CHEMISTRY, vol. 90, no. 12, 24 May 2018 (2018-05-24), pages 7230 - 7238, XP055547319, DOI: 10.1021/acs.analchem.8b00322
- TAKAYAMA MITSUO: "The Characteristics of In-source Decay in Mass Spectrometric Degradation Methods - Hydrogen-Attachment Dissociation (HAD)", JOURNAL OF THE MASS SPECTROMETRY SOCIETY OF JAPAN, vol. 50, no. 6, 1 January 2002 (2002-01-01), pages 337 - 349, XP093090866
- TAKAYAMA MITSUO: "The Characteristics of In-source Decay in Mass Spectrometric Degradation Methods ῍Hydrogen-Attachment Dissociation (HAD)῍", JOURNAL OF THE MASS SPECTROMETRY SOCIETY OF JAPAN, vol. 50, no. 6, 1 January 2002 (2002-01-01), pages 337 - 349, XP093090866

## Description

### TECHNICAL FIELD

The present invention relates to a nucleic acid structural analysis method and a nucleic acid structural analysis device.

### BACKGROUND ART

In recent years, a method with mass spectrometry has been widely used as one of nucleic acid structural analysis methods, that is, methods for determining the base sequence of DNA or RNA and specifying the type of chemical modification or the site being subjected to the chemical modification. In such analysis, MSⁿ analysis is performed on a nucleic acid to be analyzed with a mass spectrometer capable of performing MSⁿ analysis (n is an integer of 2 or more), which is mass spectrometry involving n-1 times of ion dissociations. That is, ions generated from a target nucleic acid (test nucleic acid) are dissociated by an appropriate dissociation method, and various fragment ions (also referred to as product ions) are generated from the original ions (referred to as precursor ions). These fragment ions are separated according to m/z (mass-to-charge ratio) and detected to create a mass spectrum representing m/z and detection intensity of each fragment ion.

As a method for dissociating precursor ions in the MSⁿ analysis of nucleic acids as described above, a collision induced dissociation (CID) method in which precursor ions are collided with a gas (typically an inert gas) to promote dissociation, an infrared multiphoton dissociation (IRMPD) method in which internal energy is increased by an infrared laser to promote dissociation of precursor ions, an electron transfer dissociation (ETD) method in which negative ions are collided with precursor ions, an electron capture dissociation (ECD) method in which electrons are irradiated to precursor ions, and the like are known.

### CITATION LIST

### PATENT LITERATURE

Patent Literature 1: JP 2016-223777 A
Patent Literature 2: WO 2015/133259 A

### NON PATENT LITERATURE

Non Patent Literature 1: Stefan Schurch, Mass Spectrometry Reviews, 2016, Volume35, Issue 4, pp.483-523
Non-Patent Literature 2: Santos IC and Brodbelt JS: "Recent developments in the characterization of nucleic acids by liquid chromatography, capillary electrophoresis, ion mobility, and mass spectrometry (2010-2020)", J Separation Sci, 2020, volume 44, issue 1, pp. 340-372
Non-Patent Literature 3: Mo J and Hâkansson K: "Characterization of nucleic acid higher order structure by high-resolution tandem mass spectrometry", Anal Bioanal Chem, 2006, volume 386, number 2, pp. 675-681
Non-Patent Literature 4: Takahashi H et al.: "Hydrogen Attachment/Abstraction Dissociation (HAD) of Gas-Phase Peptide Ions for Tandem Mass Spectrometry", Anal. Chem., 2016, volume 88, issue 7, pp. 3810-381
Non-Patent Literature 5: Takahashi H et al.: "Structural Analysis of Phospholipid Using Hydrogen Abstraction Dissociation and Oxygen Attachment Dissociation in Tandem Mass Spectrometry", Anal. Chem., 2018, volume 90, issue 12, pp. 7230-7238

### SUMMARY OF INVENTION

### TECHNICAL PROBLEM

When precursor ions derived from nucleic acids are dissociated by the CID method or the IRMPD method, some problems are reported in that nucleic acid bases (hereinafter, may be simply referred to as base) are eliminated from generated fragment ions (base elimination ions) (refer to, for example, Non Patent Literature 1), the sugar-phosphate skeletons of the precursor ions are dissociated at a plurality of positions to generate internal fragment ions, and chemical species having no charge are eliminated from the precursor ions to generate neutral loss ions. These base elimination ions, internal fragment ions, or neutral loss ions do not contribute to the estimation of the base sequence, and thus using the CID method or the IRMPD method is problematic in that the difficulty of estimating the base sequence of the nucleic acid by analyzing the mass spectrum is increased.

On the other hand, in the ETD method or the ECD method, the base elimination ions, the internal fragment ions, or the neutral loss ions as described above are hardly generated. However, the ETD method and the ECD method are generally effective only for positive ions, and it is difficult to dissociate negative ions. There is also a method called negative ETD (NETD) capable of dissociating negative ions, but there is a restriction that monovalent negative ions cannot be dissociated even by this method. In addition, the ETD method and the ECD method can only perform in principle dissociation of polyvalent ions. As is well known, ions generated by some ionization methods such as a matrix-assisted laser desorption/ionization (MALDI) method are mainly monovalent positive ions, so the inability to dissociate monovalent positive ions is a major limitation for analytical methods. The scientific review from Santos and Brodbelt (Non-Patent Literature 2) and the article from Mo and Hâkansson (Non-Patent Literature 3) disclose nucleic acid structural analysis methods using mass spectrometry, especially tandem mass spectrometry. Takahashi H et al. (Non-Patent Literature 4 and 5) disclose the use of HAD-MS/MS to characterize peptides, proteins or lipids but not nucleic acids.

The present invention has been made in view of such a point, and an object of the present invention is to provide a nucleic acid structural analysis method and a nucleic acid structural analysis device capable of dealing with precursor ions of various charges and easily estimating the structure of a nucleic acid based on a mass spectrum.

### SOLUTION TO PROBLEM

A nucleic acid structural analysis method according to the present invention, which has been made to solve the above problems, includes:
an HAD-MSⁿ analysis step of introducing a hydrogen radical into a space in which an ion derived from a test nucleic acid is present to perform dissociation of the ion, and performing mass spectrometry on a plurality of fragment ions generated by the dissociation to collect m/z information on the plurality of fragment ions; and
a structure estimation step of estimating a structure of the test nucleic acid on a basis of the m/z information on the plurality of fragment ions obtained in the HAD-MSⁿ analysis step.

In addition, a nucleic acid structural analysis device according to the present invention, which has been made to solve the above problems, includes:
an HAD-MSⁿ analysis execution unit configured to introduce a hydrogen radical into a space in which an ion derived from a test nucleic acid is present to perform dissociation of the ion, and to perform mass spectrometry on a plurality of fragment ions generated by the dissociation to collect m/z information on the plurality of fragment ions; and
a structure estimation unit configured to estimate a structure of the test nucleic acid on a basis of the m/z information on the plurality of fragment ions obtained in the HAD-MSn analysis execution unit.

### ADVANTAGEOUS EFFECTS OF INVENTION

The nucleic acid structural analysis method or the nucleic acid structural analysis device according to the present invention can deal with precursor ions of various charges and easily estimate the structure of a nucleic acid based on a mass spectrum.

### BRIEF DESCRIPTION OF DRAWINGS

[Fig. 1] Fig. 1 is a schematic configuration diagram of a nucleic acid structural analysis device according to an embodiment of the present invention.
[Fig. 2] Fig. 2 is a flowchart showing an example of a nucleic acid structural analysis procedure by the device.
[Fig. 3] Fig. 3 is a diagram showing a mass spectrum of fragment ions obtained by CID-MS² analysis of a nucleic acid preparation, and a base sequence of the nucleic acid preparation and each attributed series ion.
[Fig. 4] Fig. 4 is a diagram showing a mass spectrum of fragment ions obtained by HAD-MS² analysis of a nucleic acid preparation, and a base sequence of the nucleic acid preparation and each attributed series ion.
[Fig. 5] Fig. 5 is a flowchart showing another example of a nucleic acid structural analysis procedure by the device.

### DESCRIPTION OF EMBODIMENTS

The present inventors have intensively investigated a method of dissociating ions by attaching a hydrogen radical to an ion as one method of unpaired electron-induced dissociation with uncharged particles, and have already proposed a method of favorably dissociating peptide-derived ions with a hydrogen radical (refer to Patent Literature 1). This dissociation method can also dissociate, with high efficiency, monovalent ions and negative ions that cannot be dissociated by an unpaired electron-induced dissociation method with charged particles such as the ETD method or the ECD method.

In the course of repeating various experiments regarding the ion dissociation method (Hydrogen Attachment Dissociation, hereinafter referred to as "HAD") by attachment of hydrogen radical particles as described above, the present inventors have found a feature that fragment ions that do not contribute to the structure determination of a nucleic acid, such as base elimination ions, neutral loss ions, and internal fragment ions as described above, are less likely to be generated when ions derived from the nucleic acid are dissociated by HAD, and have arrived at the present invention on a basis of this finding.

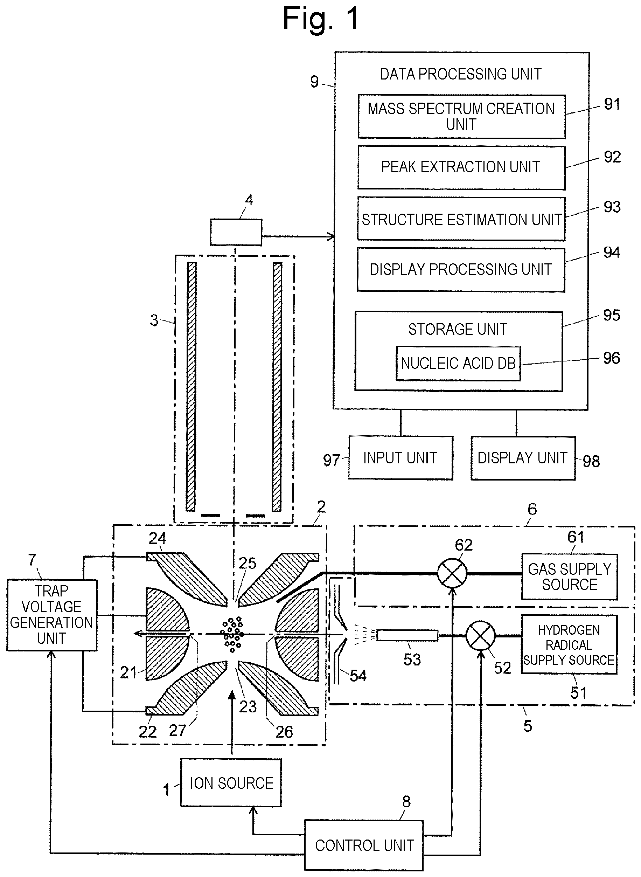

Hereinafter, an embodiment of a nucleic acid structural analysis device and a nucleic acid structural analysis method according to the present invention will be described with reference to the drawings. Fig. 1 is a schematic configuration diagram of a nucleic acid structural analysis device according to the present embodiment.

This nucleic acid structural analysis device includes a mass spectrometer, and the mass spectrometer includes an ion source 1 configured to ionize a target sample component (that is, a test nucleic acid), an ion trap 2 configured to trap ions generated by the ion source 1 by the action of a radio-frequency electric field, a time-of-flight mass separation unit 3 configured to separate ions ejected from the ion trap 2 according to m/z, and an ion detector 4 configured to detect the separated ions. In addition, the mass spectrometer includes a hydrogen radical supply unit 5 configured to supply hydrogen radicals into the ion trap 2 to dissociate ions trapped in the ion trap 2, a gas supply unit 6 configured to supply a predetermined gas into the ion trap 2, a trap voltage generation unit 7, and a control unit 8. The detection signal by the ion detector 4 is input to a data processing unit 9. The ion source 1, the ion trap 2, the time-of-flight mass separation unit 3, the ion detector 4, the hydrogen radical supply unit 5, the gas supply unit 6, the trap voltage generation unit 7, and the control unit 8 correspond to "HAD-MSⁿ analysis execution unit" in the present invention.

The control unit 8 and the data processing unit 9 are actually computers such as personal computers. The data processing unit 9 performs analysis processing characteristic of the nucleic acid structural analysis device according to the present embodiment, and includes a mass spectrum creation unit 91, a peak extraction unit 92, a structure estimation unit 93, and a display processing unit 94 as functional blocks. These functional blocks are embodied by executing a predetermined program previously installed in the computer. In addition, a storage unit 95 provided in the computer constituting the data processing unit 9 stores a nucleic acid database (nucleic acid DB) 96 in which information such as base sequences of a large number of natural or artificial known nucleic acids is recorded. Furthermore, an input unit 97 made of a keyboard and a pointing device such as a mouse, and a display unit 98 made of a liquid crystal display or the like are connected to the data processing unit 9.

The ion source 1 is, for example, an ion source using an ionization method such as a MALDI method. The ion trap 2 is a three-dimensional quadrupole ion trap including an annular ring electrode 21 and a pair of end cap electrodes 22 and 24 disposed to face each other across the ring electrode 21. In response to an instruction from the control unit 8, the trap voltage generation unit 7 applies one of a radio-frequency voltage and a direct-current voltage or a combined voltage thereof to each of the electrodes 21, 22, and 24 at a predetermined timing. The time-of-flight mass separation unit 3 is a linear type in this example, but may be a reflectron type, a multi-turn type, or the like, and may not be a time-of-flight mass separation unit. Instead of the time-of flight mass separation unit 3, for example, mass separation with by an ion separation function of the ion trap 2 itself, or an orbitrap may be used.

The hydrogen radical supply unit 5 includes a hydrogen radical supply source 51 configured to store hydrogen radicals or generate hydrogen radicals, a valve 52 configured to be capable of adjusting a flow rate, a nozzle 53 configured to eject hydrogen radicals, and a skimmer 54 configured to have an opening on a central axis of an ejection flow from the nozzle 53, to separate gases such as diffusing hydrogen molecules, and to extract a hydrogen radical flow having a small diameter.

The gas supply unit 6 includes a gas supply source 61 configured to store an inert gas such as helium gas or argon gas, and a valve 62 configured to be capable of adjusting a flow rate.

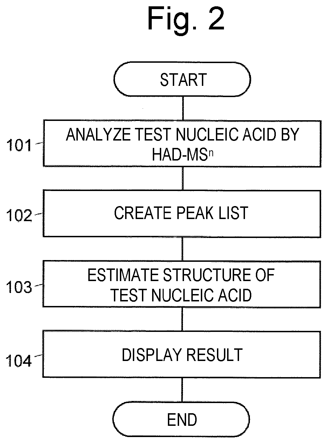

The procedure of structural analysis in the nucleic acid structural analysis device of the present embodiment will be described with reference to the flowchart of Fig. 2.

### [Step 101: HAD-MSⁿ analysis of test nucleic acid]

First, a sample containing a test nucleic acid is subjected to mass spectrometry (hereinafter, referred to as HAD-MSⁿ analysis) accompanied by ion dissociation by the HAD method. In this case, various ions generated from the sample containing the test nucleic acid in the ion source 1 are ejected from the ion source 1 in a packet shape, and introduced into the ion trap 2 through an ion introduction hole 23 formed in an inlet-side end cap electrode 22. The test nucleic acid-derived ions introduced into the ion trap 2 are trapped by a radio-frequency electric field formed in the ion trap 2 by a voltage applied from the trap voltage generation unit 7 to the ring electrode 21. Then, a predetermined voltage is applied from the trap voltage generation unit 7 to the ring electrode 21 and the like, whereby ions other than targeted ions having a specific m/z are excited and excluded from the ion trap 2. Thus, precursor ions having a specific m/z are selectively captured in the ion trap 2.

Subsequently, the valve 62 is opened in the gas supply unit 6, and an inert gas is introduced into the ion trap 2 as a cooling gas, whereby the precursor ions are cooled. Thus, the precursor ions are converged near a center of the ion trap 2.

In this state, the valve 52 of the hydrogen radical supply unit 5 is opened, and the gas containing hydrogen radicals is ejected from the nozzle 53. The gas such as the hydrogen gas is removed by the skimmer 54 located in front of the jet flow, and the hydrogen radicals having passed through the opening of the skimmer 54 become in a beam-like state having a small diameter and pass through the radical particle introduction port 26 bored in the ring electrode 21. Then, these hydrogen radicals are introduced into the ion trap 2 and react with precursor ions trapped in the ion trap 2. In this case, the opening degree of the valve 52 is adjusted such that the flow rate of hydrogen radicals introduced into the ion trap 2 becomes a predetermined flow rate (for example, 4×10¹⁰ [atoms/s]) or more. In addition, the introduction time of the hydrogen radicals is also appropriately set. The reaction with the hydrogen radical causes the precursor ions to undergo unpaired electron induced dissociation, and fragment ions derived from the precursor ions are generated. Various fragment ions generated by the dissociation of ions (that is, HAD) by the reaction with hydrogen radicals as described above are trapped in the ion trap 2, and cooling is performed.

Thereafter, a high direct-current voltage is applied from the trap voltage generation unit 7 to the end cap electrodes 22 and 24 at a predetermined timing, whereby the fragment ions trapped in the ion trap 2 receive acceleration energy and are simultaneously ejected through the ion ejection hole 25. In this manner, the fragment ions having a constant acceleration energy are introduced into a flight space of the time-of-flight mass separation unit 3, and are separated according to m/z while flying in the flight space. The ion detector 4 sequentially detects the separated fragment ions. In the data processing unit 9 that has received the detection signal from the ion detector 4, the mass spectrum creation unit 91 creates a time-of-flight spectrum in which the time of emission of the fragment ions from the ion trap 2 is time zero. Then, the time-of-flight is converted into m/z using mass calibration information which is previously obtained, whereby a mass spectrum of the fragment ions is created.

In the mass spectrum thus obtained, peaks of various fragment ions derived from the test nucleic acid are observed, but performing ion dissociation by the HAD method can suppress the generation of fragment ions that do not contribute to the structure estimation of the test nucleic acid, such as fragment ions from which a nucleic acid base has been eliminated. This will be described below with reference to an actual measurement example.

For actual measurement, a nucleic acid preparation (base sequence: 5'-GCACATTG-3', molecular weight: 2409.6) which is single-stranded DNA was used, and MS² analysis accompanied by ion dissociation by CID (that is, CID-MS² analysis) and MS² analysis accompanied by ion dissociation by HAD (that is, HAD-MS² analysis) were performed on the nucleic acid preparation. The mass spectrometer used for the measurement is a MALDI digital ion trap time-of-flight mass spectrometer (MALDI-DIT-TOF MS: manufactured by Shimadzu Corporation) equipped with a hydrogen radical particle irradiation mechanism as shown in Fig. 1. In actual measurement, the nucleic acid preparation was introduced into the ion source 1, and MS² analysis was performed using a trivalent negative ion [M-3H]³⁻ as a precursor ion. In the CID-MS² analysis, the precursor ions were dissociated by supplying the CID gas from the gas supply unit 6 to the ion trap 2, and in the HAD-MS² analysis, the precursor ions were dissociated by supplying hydrogen radicals from the hydrogen radical supply unit 5 to the ion trap 2.

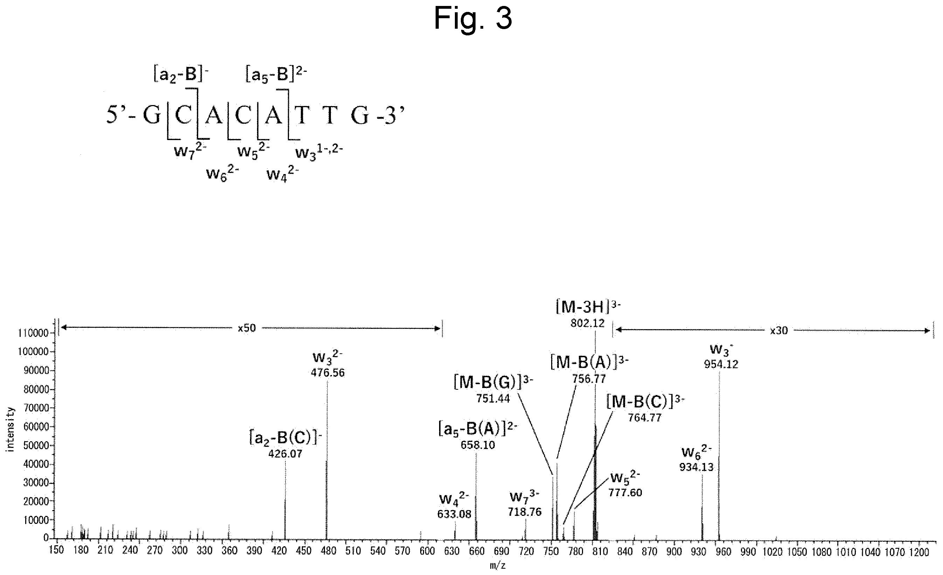

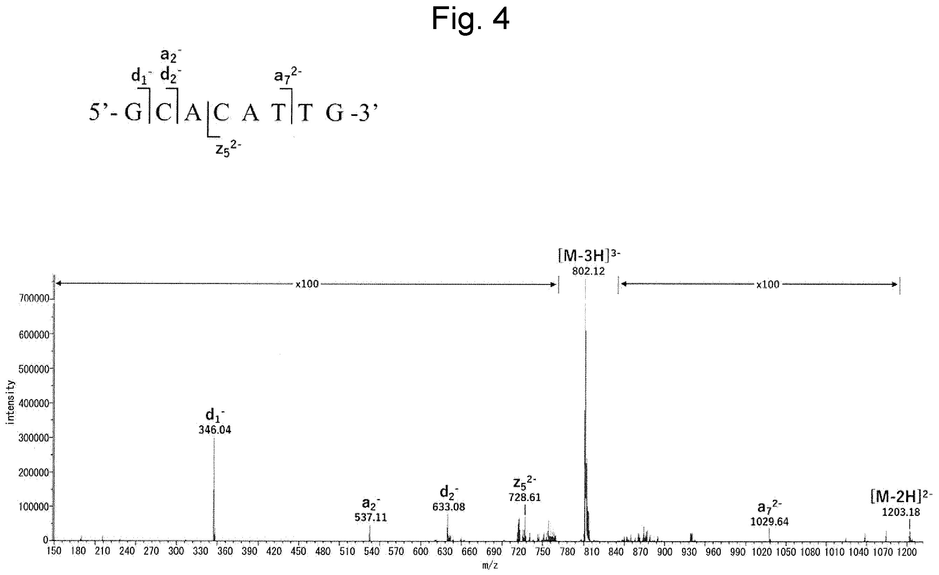

The results of the CID-MS² analysis are shown in Fig. 3, and the results of the HAD-MS² analysis are shown in Fig. 4. In Figs. 3 and 4, a base sequence of the nucleic acid preparation and an identifier representing each ion species assigned from a peak on the mass spectrum are shown in an upper part of a mass spectrum of fragment ions. In Figs. 3 and 4, each peak in the mass spectrum is denoted by m/z of the peak and an identifier representing an ion species corresponding to the peak. The identifier represents each ion species in a fragment ion series according to a nucleic acid dissociation pattern naming rule. In this naming rule, fragment ions including the 5 '-end are denoted as aₙ⁻, bₙ⁻, cₙ⁻, and dₙ⁻, and fragment ions at the 3'-end in the opposite side are denoted as wₘ⁻, xₘ⁻, yₘ⁻, and zₘ⁻. The suffixes ₙ and ₘ indicate the number of constitutional units (that is, the number of nucleotides) from the corresponding end to the dissociation site.

In the mass spectrum (CID spectrum) shown in Fig. 3, in addition to the peak of the fragment ion which can be attributed to the base sequence and in which the nucleic acid base is retained, there was observed the peak [X-B(Y)] of the fragment ion from which the nucleic acid base is eliminated, the fragment ion not being possible to be attributed to the base sequence. For example, [a₅-B(A)]²⁻ in the figure is a peak in which one nucleobase adenine (A) is eliminated from an a₅²⁻ ion, but it cannot be identified which of the two A in the nucleic acid preparation is eliminated, and thus this peak does not contribute to the estimation of the base sequence. Such a base elimination peak not only does not contribute to the estimation of the base sequence but also complicates the mass spectrum, and thus it is difficult to analyze the base sequence on a basis of the mass spectrum.

In contrast, in the mass spectrum (HAD spectrum) shown in Fig. 4, the base elimination peak as described above is not observed, and it is found that the base sequence of the test nucleic acid can be identified over a wider range than the case of using the CID spectrum. Specifically, a sequence of 3 bases on the 5' side (GCA) and a sequence of 2 bases on the 3' side (TG) can be specified from the fragment ion peak on the spectrum. Furthermore, from the m/z difference between the fragment ion z₅ and the fragment ion a₇, it can be specified that there is a combination of nucleic acid bases C, A, and T between the 3 bases on the 5' side and the 2 bases on the 3' side. The HAD spectrum in this actual measurement example cannot cover the entire base sequence of the nucleic acid preparation, but it is considered that the coverage ratio (sequence coverage) of the base sequence can be improved by increasing the types of fragment ions by adjusting the mass spectrometry conditions to increase the ion amount of precursor ions or to increase the reaction efficiency of HAD.

### [Step 102: Creation of peak list]

In the nucleic acid structural analysis device according to the present embodiment, when a mass spectrum of fragment ions is created in the mass spectrum creation unit 91, the peak extraction unit 92 extracts all peaks satisfying a predetermined condition (for example, signal intensity is equal to or greater than a predetermined threshold value) from the mass spectrum, and creates a peak list in which m/z and intensity of each peak are described. Furthermore, the peak extraction unit 92 inputs information indicating "the ion corresponding to each peak described in the peak list is a fragment ion retaining a nucleic acid base" to the structure estimation unit 93 together with the peak list.

### [Step 103: Structure estimation of test nucleic acid]

The structure estimation unit 93 that has received the information and the peak list estimates the base sequence of the test nucleic acid by database search or de novo sequencing.

When estimating the base sequence by database search, the structure estimation unit 93 creates an m/z list that is a list in which the base sequences of a plurality of fragments that can be generated from each known nucleic acid and the theoretical value (theoretical m/z) of m/z of an ion (fragment ion) corresponding to each fragment are described on a basis of the base sequence of each of a large number of known nucleic acids stored in the nucleic acid database 96, and collates the m/z list for each known nucleic acid with the peak list of the test nucleic acid.

When a test nucleic acid is analyzed by CID-MSⁿ analysis as in the related art, both fragment ions from which a nucleic acid base has been eliminated (base elimination ions) and fragment ions from which a nucleic acid base has not been eliminated (base retention ions) are generated, and thus it is necessary to perform a search in consideration of both of the above fragment ions in the database search. That is, for each of the plurality of fragments that can be generated from the known nucleic acid, it is necessary to calculate the theory m/z for both the case where the ion corresponding to the fragment is a base elimination ion and the case where the ion is a base retention ion, and to collate the m/z list describing the theory m/z with the peak list of the test nucleic acid, and thus can cause such a problem that not only the calculation amount increases but also the false answer rate increases.

In contrast, in the nucleic acid structural analysis device according to the present embodiment, the test nucleic acid is analyzed by HAD-MSⁿ analysis, and thus it can be assumed that all fragment ions generated from the test nucleic acid are base retention ions. In the device according to the present embodiment, the above-described information "the ion corresponding to each peak described in the peak list is a fragment ion retaining a nucleic acid base" is also used in addition to the peak list of the test nucleic acid when performing the database search as described above. That is, for each of a large number of known nucleic acids stored in the nucleic acid database 96, the structure estimation unit 93 calculates m/z in a case where an ion corresponding to each of a plurality of fragments that can be generated from the known nucleic acid is a base retention ion, creates an m/z list in which the base sequence of each fragment and the theory m/z of the ion corresponding thereto are described in association with each other, and collates the m/z list for each known nucleic acid with the peak list of the test nucleic acid. Then, for each of the known nucleic acids, a score is calculated, the score indicating the probability that the base sequence of the known nucleic acid is the same as the base sequence of the test nucleic acid.

Specifically, for example, the following processing is executed. First, for each peak included in the peak list of the test nucleic acid, whether or not the peak is attributable to the fragment is examined by examining whether or not m/z of the peak falls within a predetermined m/z range centered on the theoretical m/z of the fragment that can be generated from the known sequence. Then, when it is determined for all peaks in the peak list whether the peak is attributable, an attributed peak summation value obtained by summing all signal intensities of the attributable peaks is calculated, and a total peak summation value obtained by summing signal intensities of all peaks detected in the mass spectrum is calculated, and the score is calculated on a basis of a ratio between the attributed peak summation value and the total peak summation value. This score indicates the degree of coincidence between the base sequence of the known nucleic acid and the mass spectrum obtained by actually measuring the test nucleic acid, and it can be said that the base sequence of the known nucleic acid coincides more with the mass spectrum obtained by actual measurement as the score is higher. Then, the score as described above is calculated for each of the plurality of known nucleic acids stored in the nucleic acid database 96, and the base sequence of the known nucleic acid having the highest score is used as the estimation result of the base sequence of the test nucleic acid.

In addition, when the base sequence is estimated by de novo sequencing in the structure estimation unit 93, first, the composition of the test nucleic acid is estimated from m/z of the precursor ion of the test nucleic acid, and a plurality of base sequences (predicted sequences) predicted from the composition are derived. Then, for each predicted sequence, base sequences of a plurality of fragments that can be generated from the nucleic acid having the predicted sequence and a theory m/z of an ion (fragment ion) corresponding to each fragment are calculated, and a mass list is created in which the base sequence and the theory m/z are described in association with each other. In this case, in the present embodiment, it is sufficient to calculate only m/z of the base retention ions as the theory m/z, and thus the amount of calculation can be reduced. Thereafter, for each of the predicted sequences, an m/z list is created in which the base sequence of the fragment and the theory m/z of the ion corresponding thereto are described in association, and these m/z lists are collated with the peak list of the test nucleic acid. Also in this case, for example, the same score as described above is calculated for each predicted sequence, and the predicted sequence having the highest score is used as the estimation result of the base sequence of the test nucleic acid.

### [Step 104: Display of results]

When the structure estimation of the test nucleic acid is completed by the above, the display processing unit 94 displays the result of estimation of the base sequence of the test nucleic acid on the screen of the display unit 98 together with the mass spectrum of fragment ions derived from the test nucleic acid obtained in the step 101.

In the above example, an example of estimating the base sequence as the structure of the test nucleic acid has been shown, but the structure estimation device in the present embodiment may further estimate the type and the like of chemical modification such as post-transcriptional modification when the test nucleic acid contains it. In this case, in addition to (or instead of) the nucleic acid database 96, the storage unit 95 stores a modification database that registered the type of chemical modification to the nucleotide, the amount of change in m/z caused by various modifications, and information on a site in the nucleic acid that undergoes the modification when the site in the nucleic acid is known. Then, when structure estimation by database search or de novo sequencing is performed using the mass list created from the mass spectrum obtained in the step 101, structure estimation in consideration of various chemical modifications is performed with reference to the modification database. Thereby, in addition to the base sequence of the test nucleic acid, the type of chemical modification (or the type of chemical modification and the site where the modification is applied) imparted to the test nucleic acid can be estimated.

A nucleotide is composed of a sugar, a phosphate group, and a base, and thus the type of chemical modification stored in the modification database is roughly classified into a modification to a sugar, a modification to a phosphate group, and a modification to a base. Herein, the chemical modification includes both a modification generated in vivo such as post-transcriptional modification and an artificially introduced modification.

Examples of the modification to a sugar include 2'-O-methoxyethylation (2'-MOE), 2'-O-methylation (2'-OMe), and 2'-fluoroation (2'-F), which are modifications at the sugar site 2' position, and 2',4'-BNA (LNA) modification in which an oxygen atom at the sugar site 2' position and a carbon atom at the 4' position are crosslinked, O-alkylation or O-alkoxyalkylation at the sugar site 2' position, conversion from the F form at the pyrimidine sugar site 2' position to an anhydro form or an arabino form, conversion to an Abasic form, conversion to a 2'-5' phosphodiester conjugate, and conversion to a 3'→3' (5'→5') conjugate.

Examples of the modification to a phosphate group include sulfurization (S-formation), conversion from PS to PO (substitution of a sulfur atom bonded to a phosphorus atom with an oxygen atom), trichloroacetaldehyde reactant, conversion to a C-phosphonate form, ethylene phosphodiesterification, and phosphorodithioation.

Examples of the modification to a base include pseudouridination, deamination, cyanoethyl addition to thymine, methylamine addition to cytosine, isobutyl addition to guanine, and cytosine, adenine, guanine, pyrimidine, and thymine conversion products

As described above, it has been confirmed from the measurement examples shown in Figs. 3 and 4 that base elimination can be suppressed by ion dissociation by the HAD method. A base is also a chemically modifying group for a sugar and having a molecular weight of about 150. Therefore, it is predicted that, among the "modification to a sugar" described above, those in which the modifying group has a molecular weight on the order of that of a base (or those having a molecular weight smaller than that) can suppress the elimination. In addition, for the "modification to a base" described above, it is considered that elimination of the modifying group can be suppressed as long as the molecular weight of the base is not significantly increased by the modification. Furthermore, for the "modification to a phosphate group" described above, if the molecular weight of the modifying group is similar to (or smaller than) the base, it is expected that the elimination can be suppressed. In the dissociation of ions by the HAD method, it is predicted that the elimination of modification can also be suppressed in addition to the base elimination as described above, and thus, it is considered that fragment ions useful for estimating the type and site of such chemical modification can be generated by dissociating precursor ions derived from the test nucleic acid by the HAD method.

The aforementioned embodiment is merely an example of the present invention. In the ion trap mass spectrometer of the above embodiment, the ion trap has a three-dimensional quadrupole configuration, but may be a linear ion trap. In addition, the mass spectrometer is not limited to a mass spectrometer including an ion trap, and may be any mass spectrometer capable of MSⁿ analysis, and for example, a triple quadrupole mass spectrometer including quadrupole mass filters in front of and behind a collision cell can be used.

In addition, in the above description, the nucleic acid database 96 or the modified database is stored in the data processing unit 9, but the present invention is not limited thereto, and these databases may be stored in an external device connected via an interface (not illustrated) provided in a computer constituting the data processing unit 9. In addition, the nucleic acid database 96 or the modified database may be stored in a server or the like on the Internet, and a computer constituting the data processing unit 9 may be connected to the Internet via the interface to use these databases. As such a database, a database prepared previously by a public institution or a manufacturer of a mass spectrometer may be used, or for example, a database uniquely constructed by a user may be used.

The nucleic acid structural analysis device according to the present embodiment may promote dissociation of precursor ions by collisional activation in order to improve dissociation efficiency of precursor ions by the HAD method. The collisional activation is a method of supplementarily applying energy from the outside by performing the same operation as the CID method with low energy to the extent that ions are not dissociated (refer to, for example, Patent Literature 2).

In the case of promoting the dissociation by the collisional activation, an inert gas is introduced into the ion trap 2 from the gas supply unit 6 and a predetermined resonance excitation voltage is applied from the trap voltage generation unit 7 to the end cap electrodes 22 and 24 over a predetermined period before and/or after introduction of hydrogen radicals into the ion trap 2 in which precursor ions derived from the test nucleic acid are trapped, whereby the precursor ions trapped in the ion trap 2 are excited and collided with the gas. As a result, energy is applied to the precursor ion before reacting with the hydrogen radical or the precursor ion that has reacted with the hydrogen radical but is not dissociated and is present in the ion trap 2, to be able to promote the dissociation of the ion.

In addition, in the nucleic acid structural analysis device and the nucleic acid structural analysis method according to the present embodiment, after performing mass spectrometry (HAD-MSⁿ analysis) accompanied by ion dissociation by the HAD method as described above on a test nucleic acid (or before performing HAD-MSⁿ analysis), mass spectrometry (CID-MSⁿ analysis) accompanied by ion dissociation by the CID method is performed on the test nucleic acid, and the structure of the test nucleic acid may be estimated with mass spectra obtained in both analyses in a complementary manner. In this case, the ion source 1, the ion trap 2, the time-of-flight mass separation unit 3, the ion detector 4, the gas supply unit 6, the trap voltage generation unit 7, and the control unit 8 in the nucleic acid structural analysis device according to the present embodiment correspond to the "CID-MSⁿ analysis execution unit" in the present invention.

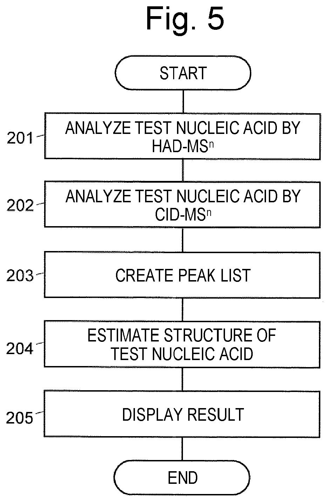

An example of a nucleic acid structural analysis procedure in this case is shown in Fig. 5. In this drawing, after the HAD-MSⁿ analysis (step 201) of the test nucleic acid is performed, the CID-MSⁿ analysis (step 202) of the test nucleic acid is performed, but the HAD-MSⁿ analysis and the CID-MSⁿ may be performed in the reverse order. In addition, in the HAD-MSⁿ analysis, the dissociation of precursor ions may be promoted by the above-described collisional activation.

In the CID-MSⁿ analysis, precursor ions having a specific m/z (having the same m/z as that of the precursor ions to be dissociated in the HAD-MSⁿ analysis) are trapped in the ion trap 2, and the valve 62 of the gas supply unit 6 is opened to introduce an inert gas as a cooling gas into the ion trap 2 to cool the precursor ions. Thereafter, a predetermined alternative current voltage is applied from the trap voltage generation unit 7 to the end cap electrodes 22 and 24 to resonantly excite the precursor ions and cause the precursor ions to collide with the inert gas (in this case, the inert gas functions as a CID gas). As a result, the precursor ions undergo energy-accumulating dissociation, and fragment ions derived from the test nucleic acid are generated. Thereafter, fragment ions in the ion trap are introduced into the flight space of the time-of-flight mass separation unit 3 at a predetermined timing, separated according to m/z, and detected by the ion detector 4.

Thereafter, the peak extraction unit 92 creates a peak list from each of the mass spectrum (HAD spectrum) obtained in the step 201 and the mass spectrum (CID spectrum) obtained in the step 202 (the step 203), and the structure estimation unit 93 estimates the structure of the test nucleic acid by database search or de novo sequencing with both peak lists (step 204). Then, the display processing unit 94 causes a display unit 98 to display the result of the structure estimation (step 205).

Collecting information on fragment ions by applying the HAD method, which is an unpaired electron-induced dissociation method, and the CID method, which is an energy accumulation-type dissociation method, to precursor ions derived from a test nucleic acid, as described above, can be expected to improve sequence coverage (that is, the proportion of the portion in which the sequence is specified in the entire base sequence of the test nucleic acid).

### [Modes]

It is understood by those skilled in the art that the plurality of exemplary embodiments described above are specific examples of the following modes.

(Clause 1) A nucleic acid structural analysis method according to one mode of the present invention is a method including:
an HAD-MSⁿ analysis step of introducing a hydrogen radical into a space in which an ion derived from a test nucleic acid is present to perform dissociation of the ion, and performing mass spectrometry on a plurality of fragment ions generated by the dissociation to collect m/z information on the plurality of fragment ions; and
a structure estimation step of estimating a structure of the test nucleic acid on a basis of the m/z information on the plurality of fragment ions obtained in the HAD-MSⁿ analysis step.

(Clause 2) The nucleic acid structural analysis method according to Clause 1 may be a method in which
in the structure estimation step, a base sequence of the test nucleic acid is estimated by de novo sequencing, and the de novo sequencing is performed on a condition that each of the plurality of fragment ions retains a nucleic acid base or a chemically modifying group for a nucleotide.

(Clause 3) The nucleic acid structural analysis method according to Clause 1 may be a method in which
in the structure estimation step, a base sequence of the test nucleic acid is estimated by database search for a nucleic acid database in which base sequences of a plurality of known nucleic acids are recorded, and the database search is performed on a condition that each of the plurality of fragment ions retains a nucleic acid base or a chemically modifying group for a nucleotide.

(Clause 4) The nucleic acid structural analysis method according to any one of Clauses 1 to 3 may be a method in which
in the HAD-MSⁿ analysis step, a neutral particle is introduced into the space before introduction of a hydrogen radical into the space, or otherwise after introduction of a hydrogen radical into the space, and an ion derived from the test nucleic acid present in the space is excited to collide with the neutral particle, to promote dissociation of the ion.

(Clause 5) The nucleic acid structural analysis method according to any one of Clauses 1 to 3 may be a method further including:
a CID-MSⁿ analysis step of introducing a neutral molecule into a space in which an ion derived from the test nucleic acid is present, performing dissociation of the ion by causing the ion to collide with the neutral molecule, and performing mass spectrometry on a plurality of fragment ions generated by the dissociation to collect m/z information on the plurality of fragment ions, in which
in the structural analysis step, a structure of the test nucleic acid is estimated on a basis of the m/z information on the plurality of fragment ions obtained in the HAD-MSⁿ analysis step and the m/z information on the plurality of fragment ions obtained in the CID-MSⁿ analysis step.

(Clause 6) The nucleic acid structural analysis method according to Clause 2 or 3 may be a method in which
a chemically modifying group for the nucleotide is a chemically modifying group for a sugar in the nucleotide.

(Clause 7) A nucleic acid structural analysis device according to one mode of the present invention is a device including:
an HAD-MSⁿ analysis execution unit configured to introduce a hydrogen radical into a space in which an ion derived from a test nucleic acid is present to perform dissociation of the ion, and to perform mass spectrometry on a plurality of fragment ions generated by the dissociation to collect m/z information of the plurality of fragment ions; and
a structure estimation unit configured to estimate a structure of the test nucleic acid on a basis of the m/z information of the plurality of fragment ions obtained in the HAD-MSⁿ analysis execution unit.

(Clause 8) The nucleic acid structural analysis device according to Clause 7 may be a device in which
a base sequence of the test nucleic acid is estimated by de novo sequencing in the structure estimation unit, and the de novo sequencing is performed on a condition that each of the plurality of fragment ions retains a nucleic acid base or a chemically modifying group for a nucleotide.

(Clause 9) The nucleic acid structural analysis device according to Clause 7 may be a device in which
a base sequence of the test nucleic acid is estimated in the structure estimation unit by database search for a nucleic acid database in which base sequences of a plurality of known nucleic acids are recorded, and the database search is performed on a condition that each of the plurality of fragment ions retains a nucleic acid base or a chemically modifying group for a nucleotide.

(Clause 10) The nucleic acid structural analysis device according to any one of Clauses 7 to 9 may be a device in which
the HAD-MSⁿ analysis unit is further configured to introduce a neutral particle into the space before introduction of a hydrogen radical into the space, or otherwise after introduction of a hydrogen radical into the space, and cause an ion derived from the test nucleic acid present in the space to be excited to collide with the neutral particle, to promote dissociation of the ion.

(Clause 11) The nucleic acid structural analysis device according to any one of Clauses 7 to 9 may be a device further including:
a CID-MSⁿ analysis unit configured to introduce a neutral molecule into a space in which an ion derived from the test nucleic acid is present to perform dissociation of the ion by causing the ion to collide with the neutral molecule, and to perform mass spectrometry on a plurality of fragment ions generated by the dissociation to collect m/z information on the plurality of fragment ions, in which
the structural analysis unit is configured to estimate a structure of the test nucleic acid on a basis of the m/z information on the plurality of fragment ions collected in the HAD-MSⁿ analysis unit and the m/z information on the plurality of fragment ions collected in the CID-MSⁿ analysis unit.

(Clause 12) The nucleic acid structural analysis device according to Clause 8 or 9 may be a device in which
a chemically modifying group for the nucleotide is a chemically modifying group for a sugar in the nucleotide.

The nucleic acid structural analysis method according to Clause 1 or the nucleic acid structural analysis device according to Clause 7 can deal with precursor ions of various charges and easily perform the structure estimation of the nucleic acid based on the mass spectrum.

The nucleic acid structural analysis method according to Clause 2 or the nucleic acid structural analysis device according to Clause 8 can estimate the structure of the test nucleic acid by the database search without considering the case where each of the plurality of fragment ions is a base elimination ion or an ion from which a chemically modifying group has been eliminated, and thus, it is possible to suppress the amount of calculation in the structure estimation.

The nucleic acid structural analysis method according to Clause 3 or the nucleic acid structural analysis device according to Clause 9 can estimate the structure of the test nucleic acid by the de novo sequencing without considering the case where each of the plurality of fragment ions is a base elimination ion or an ion from which a chemically modifying group has been eliminated, and thus, it is possible to suppress the amount of calculation in the structure estimation.

The nucleic acid structural analysis method according to Clause 4 or the nucleic acid structural analysis device according to Clause 10 can promote dissociation of precursor ions in HAD-MSⁿ analysis, and thus the SN ratio of the analysis is improved to be able to improve accuracy of structure estimation.

The nucleic acid structural analysis method according to Clause 5 or the nucleic acid structural analysis device according to Clause 11 can improve sequence coverage in structure estimation.

### REFERENCE SIGNS LIST

1... Ion Source
2... Ion Trap
3... Time-of-Flight Mass Separation Unit
4... Ion Detector
5... Hydrogen Radical Supply Unit
6... Gas Supply Unit
7... Trap Voltage Generation Unit
8... Control Unit
9... Data Processing Unit
91... Mass Spectrum Creation Unit
92... Peak Extraction Unit
93... Structure Estimation Unit
96... Nucleic Acid Database

## Claims

1. A nucleic acid structural analysis method comprising:
an HAD-MSⁿ analysis step of introducing a hydrogen radical into a space in which an ion derived from a test nucleic acid is present to perform dissociation of the ion, and performing mass spectrometry on a plurality of fragment ions generated by the dissociation to collect m/z information on the plurality of fragment ions; and
a structure estimation step of estimating a structure of the test nucleic acid on a basis of the m/z information on the plurality of fragment ions obtained in the HAD-MSⁿ analysis step.

2. The nucleic acid structural analysis method according to claim 1, wherein in the structure estimation step, a base sequence of the test nucleic acid is estimated by de novo sequencing, and the de novo sequencing is performed on a condition that each of the plurality of fragment ions retains a nucleic acid base or a chemically modifying group for a nucleotide.

3. The nucleic acid structural analysis method according to claim 1, wherein a base sequence of the test nucleic acid is estimated in the structure estimation step by database search for a nucleic acid database in which base sequences of a plurality of known nucleic acids are recorded, and the database search is performed on a condition that each of the plurality of fragment ions retains a nucleic acid base or a chemically modifying group for a nucleotide.

4. The nucleic acid structural analysis method according to claim 1, wherein in the HAD-MSⁿ analysis step, a neutral particle is introduced into the space before introduction of a hydrogen radical into the space, or otherwise after introduction of a hydrogen radical into the space, and an ion derived from the test nucleic acid present in the space is excited to collide with the neutral particle, to promote dissociation of the ion.

5. The nucleic acid structural analysis method according to claim 1, further comprising:
a CID-MSⁿ analysis step of introducing a neutral molecule into a space in which an ion derived from the test nucleic acid is present, performing dissociation of the ion by causing the ion to collide with the neutral molecule, and performing mass spectrometry on a plurality of fragment ions generated by the dissociation to collect m/z information on the plurality of fragment ions, wherein
in the structural analysis step, a structure of the test nucleic acid is estimated on a basis of the m/z information on the plurality of fragment ions obtained in the HAD-MSⁿ analysis step and the m/z information on the plurality of fragment ions obtained in the CID-MSⁿ analysis step.

6. The nucleic acid structural analysis method according to claim 2, wherein a chemically modifying group for the nucleotide is a chemically modifying group for a sugar in the nucleotide.

7. A nucleic acid structural analysis device comprising:
an HAD-MSⁿ analysis execution unit configured to introduce a hydrogen radical into a space in which an ion derived from a test nucleic acid is present to perform dissociation of the ion, and to perform mass spectrometry on a plurality of fragment ions generated by the dissociation to collect m/z information on the plurality of fragment ions; and
a structure estimation unit configured to estimate a structure of the test nucleic acid on a basis of the m/z information on the plurality of fragment ions obtained in the HAD-MSⁿ analysis execution unit.

8. The nucleic acid structural analysis device according to claim 7, wherein a base sequence of the test nucleic acid is estimated by de novo sequencing in the structure estimation unit, and the de novo sequencing is performed on a condition that each of the plurality of fragment ions retains a nucleic acid base or a chemically modifying group for a nucleotide.

9. The nucleic acid structural analysis device according to claim 7, wherein a base sequence of the test nucleic acid is estimated in the structure estimation unit by database search for a nucleic acid database in which base sequences of a plurality of known nucleic acids are recorded, and the database search is performed on a condition that each of the plurality of fragment ions retains a nucleic acid base or a chemically modifying group for a nucleotide.

10. The nucleic acid structural analysis device according to claim 7, wherein the HAD-MSⁿ analysis unit is further configured to introduce a neutral particle into the space before introduction of a hydrogen radical into the space, or otherwise after introduction of a hydrogen radical into the space, and cause an ion derived from the test nucleic acid present in the space to be excited to collide with the neutral particle, to promote dissociation of the ion.

11. The nucleic acid structural analysis device according to claim 7, further comprising:
a CID-MSⁿ analysis unit configured to introduce a neutral molecule into a space in which an ion derived from the test nucleic acid is present to perform dissociation of the ion by causing the ion to collide with the neutral molecule, and to perform mass spectrometry on a plurality of fragment ions generated by the dissociation to collect m/z information on the plurality of fragment ions, wherein
the structural analysis unit is configured to estimate a structure of the test nucleic acid on a basis of the m/z information on the plurality of fragment ions collected in the HAD-MSⁿ analysis unit and the m/z information on the plurality of fragment ions collected in the CID-MSⁿ analysis unit.

12. The nucleic acid structural analysis device according to claim 8, wherein a chemically modifying group for the nucleotide is a chemically modifying group for a sugar in the nucleotide.

13. The nucleic acid structural analysis method according to claim 1, wherein the space is a space in which a precursor ion which is an ion having a specific m/z is selectively captured out of ions derived from the test nucleic acid.

14. The nucleic acid structural analysis device according to claim 7, wherein the space is a space in which a precursor ion which is an ion having a specific m/z is selectively captured out of ions derived from the test nucleic acid.

## Patentansprüche

1. Nukleinsäurestruktur-Analyseverfahren, umfassend:
einen HAD-MSⁿ-Analyseschritt des Einführens eines Wasserstoffradikals in einen Raum, in dem ein Ion vorliegt, das von einer Prüf-Nukleinsäure abgeleitet ist, zur Durchführung einer Dissoziation des Ions, und des Durchführens einer Massenspektrometrie mit einer Mehrzahl von Fragmentionen, die durch die Dissoziation erzeugt worden sind, zum Sammeln von m/z-Informationen über die Mehrzahl von Fragmentionen; und
einen Strukturabschätzungsschritt des Abschätzens einer Struktur der Prüf-Nukleinsäure auf einer Basis der m/z-Informationen über die Mehrzahl von Fragmentionen, die in dem HAD-MSⁿ-Analyseschritt erhalten worden sind.

2. Nukleinsäurestruktur-Analyseverfahren nach Anspruch 1, wobei in dem Strukturabschätzungsschritt eine Basensequenz der Prüf-Nukleinsäure durch eine De-Novo-Sequenzierung abgeschätzt wird und die De-Novo-Sequenzierung unter der Bedingung durchgeführt wird, dass jedes der Mehrzahl von Fragmentionen eine Nukleinsäurebase oder eine chemisch modifizierende Gruppe für ein Nukleotid beibehält.

3. Nukleinsäurestruktur-Analyseverfahren nach Anspruch 1, wobei eine Basensequenz der Prüf-Nukleinsäure in dem Strukturabschätzungsschritt durch eine Datenbanksuche für eine Nukleinsäure-Datenbank abgeschätzt wird, in der Basensequenzen einer Mehrzahl von bekannten Nukleinsäuren aufgezeichnet sind, und die Datenbanksuche unter der Bedingung durchgeführt wird, dass jedes der Mehrzahl von Fragmentionen eine Nukleinsäurebase oder eine chemisch modifizierende Gruppe für ein Nukleotid beibehält.

4. Nukleinsäurestruktur-Analyseverfahren nach Anspruch 1, wobei in dem HAD-MSⁿ-Analyseschritt ein neutrales Teilchen in den Raum eingeführt wird, bevor ein Wasserstoffradikal in den Raum eingeführt wird, oder ansonsten nach der Einführung eines Wasserstoffradikals in den Raum, und ein Ion, das von der Prüf-Nukleinsäure abgeleitet ist, die in dem Raum vorliegt, angeregt wird, so dass es mit dem neutralen Teilchen zusammenstößt, wodurch die Dissoziation des Ions gefördert wird.

5. Nukleinsäurestruktur-Analyseverfahren nach Anspruch 1, ferner umfassend:
einen CID-MSⁿ-Analyseschritt des Einführens eines neutralen Moleküls in einen Raum, in dem ein Ion vorliegt, das von der Prüf-Nukleinsäure abgeleitet ist, des Durchführens einer Dissoziation des Ions durch Bewirken, dass das Ion mit dem neutralen Molekül zusammenstößt, und des Durchführens einer Massenspektrometrie mit einer Mehrzahl von Fragmentionen, die durch die Dissoziation erzeugt worden sind, zum Sammeln von m/z-Informationen über die Mehrzahl von Fragmentionen, wobei
in dem Struktur-Analyseschritt eine Struktur der Prüf-Nukleinsäure auf einer Basis der m/z-Informationen über die Mehrzahl von Fragmentionen, die in dem HAD-MSⁿ-Analyseschritt erhalten worden sind, und der m/z-Informationen über die Mehrzahl von Fragmentionen, die in dem CID-MSⁿ-Analyseschritt erhalten worden sind, abgeschätzt wird.

6. Nukleinsäurestruktur-Analyseverfahren nach Anspruch 2, wobei eine chemisch modifizierende Gruppe für das Nukleotid eine chemisch modifizierende Gruppe für einen Zucker in dem Nukleotid ist.

7. Nukleinsäurestruktur-Analysevorrichtung, umfassend:
eine HAD-MSⁿ-Analyseausführungseinheit, die zum Einführen eines Wasserstoffradikals in einen Raum, in dem ein Ion vorliegt, das von einer Prüf-Nukleinsäure abgeleitet ist, zur Durchführung einer Dissoziation des Ions, und zum Durchführen einer Massenspektrometrie mit einer Mehrzahl von Fragmentionen, die durch die Dissoziation erzeugt worden sind, zum Sammeln von m/z-Informationen über die Mehrzahl von Fragmentionen ausgebildet ist; und
eine Strukturabschätzungseinheit, die zum Abschätzen einer Struktur der Prüf-Nukleinsäure auf einer Basis der m/z-Informationen über die Mehrzahl von Fragmentionen, die in der HAD-MSⁿ-Analyseausführungseinheit erhalten worden sind, ausgebildet ist.

8. Nukleinsäurestruktur-Analysevorrichtung nach Anspruch 7, wobei eine Basensequenz der Prüf-Nukleinsäure durch eine De-Novo-Sequenzierung in der Strukturabschätzungseinheit abgeschätzt wird und die De-Novo-Sequenzierung unter der Bedingung durchgeführt wird, dass jedes der Mehrzahl von Fragmentionen eine Nukleinsäurebase oder eine chemisch modifizierende Gruppe für ein Nukleotid beibehält.

9. Nukleinsäurestruktur-Analysevorrichtung nach Anspruch 7, wobei eine Basensequenz der Prüf-Nukleinsäure in dem Strukturabschätzungsschritt durch eine Datenbanksuche für eine Nukleinsäure-Datenbank abgeschätzt wird, in der Basensequenzen einer Mehrzahl von bekannten Nukleinsäuren aufgezeichnet sind, und die Datenbanksuche unter der Bedingung durchgeführt wird, dass jedes der Mehrzahl von Fragmentionen eine Nukleinsäurebase oder eine chemisch modifizierende Gruppe für ein Nukleotid beibehält.

10. Nukleinsäurestruktur-Analysevorrichtung nach Anspruch 7, wobei die HAD-MSⁿ-Analyseeinheit ferner zum Einführen eines neutralen Teilchens in den Raum, bevor ein Wasserstoffradikal in den Raum eingeführt wird, oder ansonsten nach der Einführung eines Wasserstoffradikals in den Raum, und zum Bewirken, dass ein Ion, das von der Prüf-Nukleinsäure abgeleitet ist, die in dem Raum vorliegt, angeregt wird, so dass es mit dem neutralen Teilchen zusammenstößt, wodurch die Dissoziation des Ions gefördert wird, ausgebildet ist.

11. Nukleinsäurestruktur-Analysevorrichtung nach Anspruch 7, ferner umfassend:
eine CID-MSⁿ-Analyseeinheit, die zum Einführen eines neutralen Moleküls in einen Raum, in dem ein Ion vorliegt, das von der Prüf-Nukleinsäure abgeleitet ist, zur Durchführung einer Dissoziation des Ions durch Bewirken, dass das Ion mit dem neutralen Molekül zusammenstößt, und zum Durchführen einer Massenspektrometrie mit einer Mehrzahl von Fragmentionen, die durch die Dissoziation erzeugt worden sind, zum Sammeln von m/z-Informationen über die Mehrzahl von Fragmentionen ausgebildet ist, wobei
die Struktur-Analyseeinheit zum Abschätzen einer Struktur der Prüf-Nukleinsäure auf einer Basis der m/z-Informationen über die Mehrzahl von Fragmentionen, die in der HAD-MSⁿ-Analyseeinheit gesammelt worden sind, und der m/z-Informationen über die Mehrzahl von Fragmentionen, die in der CID-MSⁿ-Analyseseinheit gesammelt worden sind, ausgebildet ist.

12. Nukleinsäurestruktur-Analysevorrichtung nach Anspruch 8, wobei eine chemisch modifizierende Gruppe für das Nukleotid eine chemisch modifizierende Gruppe für einen Zucker in dem Nukleotid ist.

13. Nukleinsäurestruktur-Analyseverfahren nach Anspruch 1, wobei der Raum ein Raum ist, in dem ein Vorstufenion, das ein Ion mit einem spezifischen m/z ist, von Ionen, die von der Prüf-Nukleinsäure abgeleitet sind, selektiv eingefangen wird.

14. Nukleinsäurestruktur-Analysevorrichtung nach Anspruch 7, wobei der Raum ein Raum ist, in dem ein Vorstufenion, das ein Ion mit einem spezifischen m/z ist, von Ionen, die von der Prüf-Nukleinsäure abgeleitet sind, selektiv eingefangen wird.

## Revendications

1. Procédé d'analyse structurelle d'acide nucléique comprenant :
une étape d'analyse HAD-MSⁿ consistant à introduire un radical d'hydrogène dans un espace dans lequel un ion dérivé d'un acide nucléique test est présent pour réaliser une dissociation de l'ion, et réaliser une spectrométrie de masse sur une pluralité d'ions fragmentés générés par la dissociation pour recueillir des informations m/z sur la pluralité d'ions fragmentés ; et
une étape d'estimation de structure consistant à estimer une structure de l'acide nucléique test en fonction des informations m/z sur la pluralité d'ions fragmentés obtenus dans l'étape d'analyse HAD-MSⁿ.

2. Procédé d'analyse structurelle d'acide nucléique selon la revendication 1, dans lequel dans l'étape d'estimation de structure, une séquence de bases de l'acide nucléique test est estimée par séquençage de novo, et le séquençage de novo est réalisé à une condition selon laquelle chacun de la pluralité d'ions fragmentés conserve une base d'acide nucléique ou un groupe de modification chimique pour un nucléotide.

3. Procédé d'analyse structurelle d'acide nucléique selon la revendication 1, dans lequel une séquence de bases de l'acide nucléique test est estimée dans l'étape d'estimation de structure par recherche de base de données pour une base de données d'acides nucléiques dans laquelle des séquences de bases d'une pluralité d'acides nucléiques connus sont enregistrées, et la recherche de base de données est réalisée à une condition selon laquelle chacun de la pluralité d'ions fragmentés conserve une base d'acide nucléique ou un groupe de modification chimique pour un nucléotide.

4. Procédé d'analyse structurelle d'acide nucléique selon la revendication 1, dans lequel dans l'étape d'analyse HAD-MSⁿ, une particule neutre est introduite dans l'espace avant introduction d'un radical d'hydrogène dans l'espace, ou autrement après introduction d'un radical d'hydrogène dans l'espace, et un ion dérivé de l'acide nucléique test présent dans l'espace est excité pour entrer en collision avec la particule neutre pour promouvoir la dissociation de l'ion.

5. Procédé d'analyse structurelle d'acide nucléique selon la revendication 1, comprenant en outre :
une étape d'analyse CID-MSⁿ consistant à introduire une molécule neutre dans un espace dans lequel un ion dérivé de l'acide nucléique test est présent, réaliser une dissociation de l'ion en amenant l'ion à entrer en collision avec la molécule neutre, et réaliser une spectrométrie de masse sur une pluralité d'ions fragmentés générés par la dissociation pour recueillir des informations m/z sur la pluralité d'ions fragmentés, dans lequel
dans l'étape d'analyse structurelle, une structure de l'acide nucléique test est estimée en fonction des informations m/z sur la pluralité d'ions fragmentés obtenus dans l'étape d'analyse HAD-MSⁿ et des informations m/z sur la pluralité d'ions fragmentés obtenus dans l'étape d'analyse CID-MSⁿ.

6. Procédé d'analyse structurelle d'acide nucléique selon la revendication 2, dans lequel un groupe de modification chimique pour le nucléotide est un groupe de modification chimique pour un sucre dans le nucléotide.

7. Dispositif d'analyse structurelle d'acide nucléique comprenant :
un module d'exécution d'analyse HAD-MSⁿ configuré pour introduire un radical d'hydrogène dans un espace dans lequel un ion dérivé d'un acide nucléique test est présent pour réaliser une dissociation de l'ion, et pour réaliser une spectrométrie de masse sur une pluralité d'ions fragmentés générés par la dissociation pour recueillir des informations m/z sur la pluralité d'ions fragmentés ; et
un module d'estimation de structure configuré pour estimer une structure de l'acide nucléique test en fonction des informations m/z sur la pluralité d'ions fragmentés obtenus dans le module d'exécution d'analyse HAD-MSⁿ.

8. Dispositif d'analyse structurelle d'acide nucléique selon la revendication 7, dans lequel une séquence de bases de l'acide nucléique test est estimée par séquençage de novo dans le module d'estimation de structure, et le séquençage de novo est réalisé à une condition selon laquelle chacun de la pluralité d'ions fragmentés conserve une base d'acide nucléique ou un groupe de modification chimique pour un nucléotide.

9. Dispositif d'analyse structurelle d'acide nucléique selon la revendication 7, dans lequel une séquence de bases de l'acide nucléique test est estimée dans le module d'estimation de structure par recherche de base de données pour une base de données d'acides nucléiques dans laquelle des séquences de bases d'une pluralité d'acides nucléiques connus sont enregistrées, et la recherche de base de données est réalisée à une condition selon laquelle chacun de la pluralité d'ions fragmentés conserve une base d'acide nucléique ou un groupe de modification chimique pour un nucléotide.

10. Dispositif d'analyse structurelle d'acide nucléique selon la revendication 7, dans lequel le module d'analyse HAD-MSⁿ est en outre configuré pour introduire une particule neutre dans l'espace avant introduction d'un radical d'hydrogène dans l'espace, ou autrement après introduction d'un radical d'hydrogène dans l'espace, et amener un ion dérivé de l'acide nucléique test présent dans l'espace à être excité pour entrer en collision avec la particule neutre pour promouvoir la dissociation de l'ion.

11. Dispositif d'analyse structurelle d'acide nucléique selon la revendication 7, comprenant en outre :
un module d'analyse CID-MSⁿ configuré pour introduire une molécule neutre dans un espace dans lequel un ion dérivé de l'acide nucléique test est présent, pour réaliser une dissociation de l'ion en amenant l'ion à entrer en collision avec la molécule neutre, et pour réaliser une spectrométrie de masse sur une pluralité d'ions fragmentés générés par la dissociation pour recueillir des informations m/z sur la pluralité d'ions fragmentés, dans lequel
le module d'analyse structurelle est configuré pour estimer une structure de l'acide nucléique test en fonction des informations m/z sur la pluralité d'ions fragmentés recueillis dans le module d'analyse HAD-MSⁿ et des informations m/z sur la pluralité d'ions fragmentés recueillis dans le module d'analyse CID-MS".

12. Dispositif d'analyse structurelle d'acide nucléique selon la revendication 8, dans lequel un groupe de modification chimique pour le nucléotide est un groupe de modification chimique pour un sucre dans le nucléotide.

13. Procédé d'analyse structurelle d'acide nucléique selon la revendication 1, dans lequel l'espace est un espace dans lequel un ion précurseur qui est un ion ayant un m/z spécifique est capturé sélectivement parmi des ions dérivés de l'acide nucléique test.

14. Dispositif d'analyse structurelle d'acide nucléique selon la revendication 7, dans lequel l'espace est un espace dans lequel un ion précurseur qui est un ion ayant un m/z spécifique est capturé sélectivement parmi des ions dérivés de l'acide nucléique test.
